(19) Europäisches Patentamt
European Patent Office
Office européen des brevets

(11) Veröffentlichungsnummer: **0 082 486**
**B1**

# (12) EUROPÄISCHE PATENTSCHRIFT

(45) Veröffentlichungstag der Patentschrift: **06.08.86**

(51) Int. Cl.⁴: **C 12 Q 1/00**

(21) Anmeldenummer: **82111674.6**

(22) Anmeldetag: **16.12.82**

## (54) Verfahren zur kinetischen Prozesskontrolle von enzymatischen Stoffumsetzungen und Vorrichtung.

(30) Priorität: **18.12.81 DE 3150112**

(43) Veröffentlichungstag der Anmeldung: **29.06.83 Patentblatt 83/26**

(45) Bekanntmachung des Hinweises auf die Patenterteilung: **06.08.86 Patentblatt 86/32**

(84) Benannte Vertragsstaaten: **AT CH DE FR GB IT LI NL**

(56) Entgegenhaltungen:
**EP - A - 0 030 195**
**DE - A - 2 008 842**
**FR - A - 2 238 713**

**BIOTECHNOLOGY AND BIOENGINEERING, Band, XII, 1970, pages 615-631, John Wiley & Sons, Inc., T.A. BUTTERWORTH et al.: "Application of Ultrafiltration for Enzyme retention during continuous enzymatic reaction"**
**CLINICAL CHEMISTRY; Band 26, Nr. 9, August 1980, Seiten 1291-1297, Winston-Salem, USA J.F. DOOLEY et al.: "A new kinetic determination of serum 5'-nucleotidase activity, with modifications for a centrifugal analyzer"**

(73) Patentinhaber: **Gesellschaft für Biotechnologische Forschung mbH (GBF), Mascheroder Weg 1, D-3300 Braunschweig-Stöckheim (DE)**

(72) Erfinder: **Kroner, Karl-Heinz, Schneekoppeweg 30, D-3340 Wolfenbüttel (DE)**

(74) Vertreter: **Boeters, Hans Dietrich, Dr. et al, Boeters, Bauer & Partner Thomas-Wimmer-Ring 14, D-8000 München 22 (DE)**

**EP 0 082 486 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein neues Verfahren zur direkten Messung der Kinetik des enzymatischen Abbaues von hochpolymeren Substraten.

Bisher wurden für die Bestimmung von Enzym-Aktivitäten zur Kontrolle von Reaktionsabläufen sog. Endpunktsmethoden, z.B. Automat. Anal. Chem. Technicon Symp., 1967, Vol 1, S. 587ff. oder Analyt. Biochem. 23 (1968), S. 466ff. beschrieben. Bei diesen Verfahren werden die im Hydrolysereaktionsansatz gespaltenen Produkte von den nicht-umgesetzten Substraten durch nach Grösse klassierende Methoden, wie Filtration, Zentrifugation oder Dialyse abgetrennt und anschliessend die Konzentration der Spaltprodukte, z.B. photocolorimetrisch bestimmt. Diese sog. Endpunktmethoden haben den Nachteil, dass sie eine direkte Verfolgung der Reaktion nicht ermöglichen. Weitere Nachweismethoden für den Abbau von hochpolymeren Substraten beruhen darauf, dass die bei fortschreitender Reaktion eintretende Verringerung der Viskosität oder Veränderung der Lichtstreuung gemessen wird. (Vgl. Anal. Biochem., 90 (1978) S. 192ff.; Unters. Forsch., 162 (1976), S. 31ff.). Diese Verfahren sind jedoch meist nicht allgemein auf Enzymklassen anwendbar, sondern müssen je nach Enzymart von Fall zu Fall adaptiert werden, da beispielsweise bekannte Substrate unter den Messbedingungen nicht löslich sind oder die Reaktionsgeschwindigkeit oft erheblich reduziert wird. Häufig ist die Nachweisgrenze gering. Die in der Literatur beschriebenen Verfahren sind sehr zeit- und personalaufwendig, da sie im allgemeinen mehrere verschiedene Arbeitsschritte (Inkubation, Neutralisation, Fällung, Filtration, Zentrifugation, Dialyse, Anfärbung, Detektion und Auswertung) erfordern, welche zumeist nacheinander erfolgen müssen. Diese Verfahren sind zwar prinzipiell automatisierbar, aber aufgrund der Verknüpfung verschiedener heterogener Arbeitsschritte erfordern die Messgeräte einen sehr komplizierten Aufbau.

Diese Nachteile der bekannten Messmethoden von Enzymaktivitäten sollen durch das erfindungsgemässe Verfahren vermieden werden.

Ferner ist es bekannt, bei der Herstellung von enzymatischen Umsetzungsprodukten niedermolekularer biologischer Stoffe (DE-A-2 008 842 und FR-A-2 238 713) das Enzym nach der Umsetzung durch Ultrafiltration vom Reaktionsgemisch abzutrennen und es kostensparend wieder zu verwenden, wobei die Tatsache ausgenützt wird, dass alle Enzyme sich von den in Frage kommenden Hilfsstoffen und niedermolekularen Substraten, sowie den Umsetzungsprodukten so stark im Molekulargewicht unterscheiden, dass sie durch Ultrafiltration leicht von ihnen abgetrennt werden können. Wenn man diese Methode gemäss der FR-A-2 238 713 zur Konzentrationsbestimmung der niedermolekularen biologischen Stoffe anwendet, muss man jedoch berücksichtigen, dass ein geringer Anteil an nicht umgesetztem niedermolekularen Substrat bei der Ultrafiltration im Retentat verbleibt, den man bei der Wiederverwendung des Enzyms entweder durch weitere Ultrafiltrationsschritte entfernen oder rechnerisch berücksichtigen muss, was mit Hilfe eines elektronischen Rechners aus der Konzentrationsbestimmung der niedermolekularen Stoffe möglich ist, die im Reaktionsgemisch vor der Ultrafiltration oder danach im Ultrafiltrationsstrom photometrisch vorgenommen wird. Bei der Arbeitsweise gemäss der DE-A-2 008 842 und FR-A-2 238 713 bleibt also nur das eingesetzte hochmolekulare Enzym vollständig im Retentat, wohingegen die Umsetzungsprodukte sowie gegebenenfalls noch vorhandenes nicht umgesetztes Substrat gemeinsam in den Ultrafiltrationsstrom übertreten.

Gegenstand der Erfindung ist ein Verfahren zur kinetischen Prozesskontrolle von enzymatischen Stoffumsetzungen, welches dadurch gekennzeichnet ist, dass man die Reaktionsmischung, die das zu hydrolysierende Substrat und die hydrolytischen Enzyme enthält, durch eine Ultrafiltrationsmembran in zwei Volumenströme aufteilt, wobei der Ultrafiltrationsstrom die Spaltprodukte und der Retentatstrom das Substrat enthält, und anschliessend die Enzymkonzentration aus der Änderung der Substrat- oder Produktkonzentration im Retentat bzw. Ultrafiltrationsstrom ermittelt. Die bei den herkömmlichen Verfahren erforderlichen verschiedenen Reaktionsschritte, wie

1. Inkubation des Enzyms mit dem Substrat für eine vorgegebene Zeit.
2. Abstoppen der Reaktion.
3. Abtrennung der Reaktionsprodukte durch z.B. Fällung mit anschliessender Filtration oder Zentrifugation bzw. über Dialysemembranen.
4. Färbereaktion (chem. Umsetzung der Spaltprodukte).
5. Messung (z.B. photometrisch).
6. Auswertung (Differenzwertbestimmung, Eichung, Berechnung)

fallen bei diesem neuen Verfahren praktisch zeitlich zusammen, so dass das Verfahren mit einem erheblichen Zeitgewinn durchführbar ist. Das neue Verfahren, das alle Vorteile von direkten kinetischen Analysenmethoden aufweist, kann mit hochmolekularen Substraten durchgeführt werden. Eine Grössenklassierung der gelösten Substrate und Abbauprodukte durch Ultrafiltration erlaubt es, den Abbau der hochmolekularen Substrate direkt und kontinuierlich zu verfolgen. Es ist wesentlich für die direkte Messung der Reaktionsgeschwindigkeit einer enzymatischen Hydrolyse, dass die verwendete Membran den zeitlichen Verlauf der Konzentrationsveränderung nicht merklich beeinflusst. Diese Bedingung ist erfüllt, wenn man Ultrafiltrationsmembrane einsetzt, deren Zeitkonstante (Flux, Druckverlust) möglichst kleiner ist als die vermutliche Reaktionsgeschwindigkeit des enzymatischen Umsatzes.

Das erfindungsgemässe Verfahren ist sehr gut automatisierbar, wobei der Vorteil darin liegt, dass der apparative Aufwand geringer ist, weil Reaktion, Inkubation, Trennung der Spaltprodukte in einem einzigen Apparat (UF-Reaktor oder -Messzelle) kontinuierlich ablaufen und die Reaktion direkt verfolgt werden kann. Der einfache Aufbau und eine mögliche kontinuierliche Betriebsführung erlauben es, dieses Verfahren zur Prozessanalyse, z.B. an Fermentern oder Reaktoren einzusetzen. Eine Kontrolle, Steuerung und Auswertung über eine Rechnereinheit (Mikroprozessor, Prozessrechner) ist zweckmässig. Als Anwendungsgebiet kommen vor allem die Prozesskontrolle von Fermentationen oder von enzymatischen Stoffumsetzungen (Stärkeabbau, Proteinhydrolyse etc.) zur Bestimmung der enzymatischen Aktivitäten in Frage. Weiterhin ist das Verfahren im Labor, insbesondere für die Prozessanalytik bei der Aufarbeitung von Hydrolasen einsetzbar. Das neue Verfahren ermöglicht eine vollkontinuierliche automatische Betriebs- bzw. Prozesskontrolle.

Für das neue Verfahren sind handelsübliche Membranen geeignet.

Sie sollen eine optimale Trennung von Produkt und Substrat gewährleisten und hohe Standzeiten haben. Sie müssen gegen den Angriff von Enzymen und Reagenzien inert sein, möglichst geringe Adsorptionseigenschaften und ausreichende Druckfestigkeit (ca. $\Delta P$ 1 bar) haben.

Sie sollen geringe Druckverluste und eine kleine Zeitkonstante haben.

Bevorzugt werden Membranen aus Polysulfonen (Polyelektrolytkomplexe) oder aus Polyamid, welche sich besonders durch hohe Fluxraten, geringe unspezifische Adsorption und kleine Zeitkonstante auszeichnen. Beispielhaft dafür seien genannt die Membranen der Firma Amicon Corp., Typen der PM, XM, YM-Reihe mit verschiedenen Ausschlussgrenzen (5.000, 10.000, 30.000 dalton) oder der Firma Millipore der Type PTGC oder von Berghof die Type BM.

Als Messzellen eignen sich erfindungsgemäss grundsätzlich alle im Handel befindlichen kleinen Ultrafiltrationszellen oder Dialysezellen. Gut bewährt hat sich z.B. die Spiralultrafiltrationszelle, Typ CEC-1 der Firma Amicon Corp. Diese Zelle zeichnet sich durch ein geringes Totvolumen und durch eine Verminderung von Abscheidungen auf dem Filter durch Stromführung aus. Weitere Beispiele für geeignete Messzellen sind Magnetkern gerührte Mini-Reaktoren und andere als Flachmembranen verwendete Systeme. Die Abmessungen der Zellen werden im wesentlichen durch das Verhältnis des Totvolumens (Retentatvolumen) zu wirksamer Membranfläche ($V_t/F_m$) bestimmt. Am günstigsten ist ein Verhältnis $V_t/F_m \leq 1$.

Das neue Verfahren ist für die Bestimmung der Reaktivität aller Arten von Hydrolasen, wie Proteasen, Peptidasen, Glycosidasen, Phosphatasen, Lipasen oder Nucleasen und für natürliche oder chemisch modifizierte oder synthetische hochmolekulare Substrate wie Proteine, Polysaccharide oder Nucleinsäuren einsetzbar. Grundsätzlich ist auch der Einsatz für niedermolekulare Verbindungen, wie Peptide, oligomere Zucker, Dissacharide, Nucleotide oder Ester möglich. Bei Einsatz von niedermolekularen Substraten werden bevorzugt RO-Membranen (RO = reverse osmosis) oder ED-Membranen (ED = electrodialysis) verwendet.

Zur Erläuterung der Erfindung ist eine Messanordnung zur Durchführung des neuen Verfahrens in Figur 1 dargestellt. Diese Vorrichtung ist durch ein Reaktionsgefäss (5), das über eine Leitung, in der eine Pumpe (1), sowie Einrichtungen zur Regelung von Druck, Temperatur und Durchfluss (6, 7, 8) angeordnet sind, mit einer Ultrafiltrationszelle (2) verbunden ist, und zwei Leitungen, wobei zumindest in einer ein Detektor (3) angeordnet ist, von der Ultrafiltrationszelle (2) zum Reaktionsgefäss (5) gekennzeichnet ist. Die Ultrafiltrationszelle (2) ist mit dem Detektor (3) verbunden und letzterer mit dem Schreiber (4).

Die Veränderung der Produkt- oder Substratkonzentration misst man vorzugsweise kontinuierlich mit einem Spektralphotometer, Refraktometer oder Polarimeter.

Das Verfahren kann sowohl batchweise – bei automatisiertem Betrieb taktweise – erfolgen, als auch kontinuierlich, d.h. entweder das Substrat oder das Enzym werden fortlaufend durch die Zelle gepumpt und Enzym bzw. Substrat pulsweise zugegeben, resp. beide werden kontinuierlich zugegeben und gemischt. Bei der Detektion kann gegen eine Referenzzelle gemessen werden, z.B. mit einem Enzym- oder Substratstandard. Alternativ ist auch die Detektion im Retentatstrom möglich. Der vom Detektor erfasste Signalwert-Verlauf wird per Schreiber aufgezeichnet. Die Auswertung erfolgt in der Weise, dass eine Tangente an die Kurve des Messverlaufs im Punkte der max. Änderung über der Zeit angelegt wird. Die Steigung dieser Tangente ($\Delta$ S/min.) ist ein Mass für die Reaktionsgeschwindigkeit.

Die Ermittlung der Steigung kann z.B. mit einem Rechner erfolgen. Die Enzymaktivität errechnet sich nach folgender allgemeiner Formel:

$$\text{Enzymaktivität} \left[\frac{\text{units}}{\text{ml}\cdot}\right] = \frac{\Delta S \cdot \text{Verd.}}{\text{min.} \cdot E_F}$$

Verd. = Verdünnungsfaktor
$E_F$ = Eichfaktor
$\Delta S$ = Signalwert

S entspricht z.B. bei photometrischer Messung der Extinktion E, bei refraktometrischer Messung dem Lichtbrechungswinkel $\alpha$.

In der Praxis erfolgt die Ermittlung der Enzymaktivität mittels Eichkurven.

Die Eichung kann sowohl gegen das Produkt als auch gegen das Substrat vorgenommen werden. Im einfachsten Falle ist eine Eichung gegen einen definierten Enzymstandard möglich.

Beispiel 1
Bestimmung einer sauren Protease (SP-1 Fa. Henkel):
Betriebsbedingungen
UF-Zelle-CEC-1 (Amicon)
Membran-PM 30, ∅ 90 mm (Amicon)
Flow – 5 ml/min. (Rt : UF = 1:1)

| | |
|---|---|
| Reaktionsvolumen | 3.5 ml |
| Totvolumen | 3.7 ml |
| Signaltotvolumen | 2.1 ml |
| Schreiber | 500 mm/h |
| Photometer | 0.5 E/280 nm ≙ 100 % |
| Temperatur | 40 °C |

Messanordnung:
Standard-Batchbetrieb, Detektion im UF (vgl. Fig. 1)

Messlösung:
0.70 mTU/ml Fermenterprobe (70 h)

Durchführung:
Zuerst wurde die gesamte Anlage mit Puffer gespült, bis ein konstanter Wert am Photometer sich eingestellt hat. In die temperierte Reaktionsvorlage wurden dann 2.3 ml Protease Puffer (pH 3.5) und 0.2 ml Enzymlösung einpipettiert und gemischt. Diese Mischung wurde für ca. 5 min. durch den Reaktor (UF-Zelle) gepumpt, dabei stellte sich am Photometer ein dem Nullwert entsprechender Signalwert ein. Wenn dieser Signalwert konstant war, wurde in die Reaktionsvorlage 1 ml Standardcaseinlösung dazupipettiert. Damit wurde die Reaktion gestartet. Nach einer Totzeit von ca. 1 min. war die Reaktion auf dem angeschlossenen Schreiber als Veränderung der Extinktion über der Zeit ΔE/min. zu beobachten. Nach Abschluss des Tests wurde die Anlage mit Puffer wieder rückgespült. Dieser Ansatz wurde mit zwei verschiedenen Messzellen zu Vergleichszwecken durchgeführt.

Auswertung:
Aus dem Signalwertverlauf (Figur 2) wurde ΔE/min. graphisch ermittelt und aus einer Eichkurve der zugehörige Wert für die Enzymkonzentration entnommen, bzw. aus der Steigung der Eichgeraden im linearen Arbeitsbereich (Figur 3) berechnet.

Erstellung der Eichkurven:
Eichgerade (Fig. 3):
Aus Referenzmessung mit der Eichsubstanz AP-114, saure Protease (standardisiertes Enzym) und Caseinlösung als Substrat.
Messanordnung wie in Fig. 1
Die Eichkurven für die Messzellen 1 und 2 werden parallel aufgenommen, und zwar exakt unter den beschriebenen Bedingungen und nach der gleichen Prozedur, wie oben beschrieben.

Messzelle 1: $0.0120 \frac{\Delta E/\text{min.}}{\text{mTU/ml}}$

Messzelle 2: $0.0093 \frac{\Delta E/\text{min.}}{\text{mTU/ml}}$

Umrechnungsfaktor: $\frac{MZ1}{MZ2} = \frac{0.0120}{0.0093} = 1.29$

Ergebnisse:

| | | E/min. | mTU/ml | | E/min. | mTU/ml |
|---|---|---|---|---|---|---|
| MZ1: | a) | 0.009 | 0.75 | MZ2: | 0.006 | 0.68 |
| | b) | 0.009 | 0.75 | | 0.006 | 0.68 |
| | c) | 0.008 | 0.73 | | 0.007 | 0.78 |
| | | $\overline{X}=0.74$ | | | $\overline{X}=0.71$ | |

Abweichungen vom Soll-Wert (0.70 mTU/ml) = $1–4 \cdot 10^{-2}$ mTU/ml = 1–6% (0 = 3.5%).

Beispiel 2
Bestimmung einer alkalischen Protease (Fermentationsprobe Fa. Henkel)
Betriebsbedingungen:

| | |
|---|---|
| UF-Zelle: | CEC-1 (Amicon) |
| Membran: | PM 30, ∅ 90 mm (Amicon) |
| Flow: | 5.7 l/min. (Rt : UF = 1.5:1) |
| Reaktionsvolumen: | 5.0 ml |
| Kreislaufvolumen total: | 13 ml |
| Temperatur: | 50 °C |
| Substrat: | Casein (n. Hammarsten) [1.5 ml] |
| Enzymprobenvolumen: | 1 ml |

Messanordnung:
Batch-Kreislaufbetrieb, Detektion im UF bei 280 nm.

Messlösung:
Fermenterprobe der Fa. Henkel (Konzentrat), ca. 78 PE/ml (PE = Proteaseeinheit)

Durchführung:
analog zu Beispiel 1
Eichgerade (Fig. 4) aus Referenzmessung gegen Enzym-Standard (Maxatase) Steigung m = 0,55 (MZ2)

$MZ1 = 0.41 \frac{\Delta E/\text{min.}}{\text{mTU/ml}}$

$MZ2 = 0.55 \frac{\Delta E/\text{min.}}{\text{mTU/ml}}$

Umrechnungsfaktor = 0.75

Berechnung: $\frac{\Delta mOD/\text{min.}}{0.55} = PE/ml$ ($\Delta mOD = \Delta E$; OD = optische Dichte)

Messwerte MZ2

| Δm OD/min. | PE/ml |
|---|---|
| 42.5 | 77.3 |
| 45.0 | 81.8 |
| 50.0 | 90.9 |
| 40.0 | 72.7 |
| 50.0 | 90.9 |
| 50.0 | 90.9 |
| | $\bar{X}$ = 84.08 |

Abweichung vom Soll-Wert = 6.1 ≙ 7.7%

Beispiel 3
Bestimmung von Pullulanase
Betriebsbedingungen:

| | |
|---|---|
| UF-Zelle: | CEC-1 (Amicon) |
| Membran: | YM-5, Ø 90 mm (Amicon) |
| Flow: | 4 ml/min. ($R_t$ : UF = 2.2:1.8) |
| Reaktionsvolumen: | 6 ml |
| Kreislaufvolumen total: | 15.7 ml |
| Temperatur: | 35 °C |
| Substrat: | Pullulan (Serva) |
| Enzymprobenvolumen: | 0.4 ml |

Messanordnung:
Batch-Kreislaufbetrieb, Detektion im UF mit einem Polarimeter bei 578 nm Hg.

Messlösung:
Enzymprobe der Fa. ABM-Chemicals (Pulluzyme), ca. 18 U/ml.

Durchführung:
Zuerst wurde die Anlage mit Puffer gespült, bis sich am Polarimeter ein konstanter Wert eingestellt hat. Dieser Wert wurde zu Null gesetzt. In die temperierte Reaktionsvorlage wurden 5 ml Citratpuffer (pH 5.0) und 0.6 ml Pullulanlsg. (2 %ig) gegeben und für ca. 5 min. im Kreislauf über die Membran gepumpt. Die Anzeige des Polarimeters wurde erneut zu Null gesetzt. Dann wurde mit 0.4 ml Enzymlösung die Reaktion gestartet. Der Reaktionsverlauf wurde am Polarimeter bzw. auf dem angeschlossenen Schreiber beobachtet. Die Änderung der opt. Drehung über der Zeit t (min) stellt das Mass für die Reaktionsgeschwindigkeit dar ($\Delta \alpha_{Hg578}$/min ~ U/ml Pullulanase).

Auswertung:
Aus dem Signalwertverlauf wurde Δ α/min. durch Anlegen einer Geraden im Bereich der max. Steigung wie üblich graphisch ermittelt.

Berechnung:

$$\text{U/ml Pullulanase} = \frac{\Delta\alpha/\text{min.} \cdot \text{Verd.faktor}}{0.0857}$$

1 U/ml ≙ 1 μMol Maltotriose pro Minute (pH 5.0; 35 °C)

Figur 6 zeigt die Konzentrationsabhängigkeit der Pullulanase im UF-Test, gemessen mit Polarimeter Hg 578.

Eichfaktor:
0.0857 aus Eichkurve Maltotriose im Polarimeter bei Hg 578 nm (35 °C) (Fig. 5)

$$\left(m = \frac{0.17\ \text{grd} \cdot \text{ml}}{\text{mg}} = \frac{85.7\ \text{grd ml}}{\text{m Mol}}\ \frac{0.0857\ \text{grd ml}}{\text{Mol}}\right)$$

Ergebnisse:

| Δα/min. | Verd. faktor | U/ml |
|---|---|---|
| 0.048 | 39.25 | 21.9 |
| 0.048 | 39.25 | 21.9 |
| 0.043 | 39.25 | 19.7 |
| 0.042 | 39.25 | 19.2 |
| 0.047 | 39.25 | 21.5 |
| 0.043 | 39.25 | 19.7 |
| | $\bar{X}$ = 20.65 | |
| | S = 1.25 (± 6%) | |

Abweichung vom Soll-Wert = + 15%

Beispiel 4
Bestimmung von Amyloglucosidase (Glucoamylase)
Betriebsbedingungen:

| | |
|---|---|
| UF-Zelle: | CEC-1 (Amicon) |
| Membran: | PM 10, Ø 90 mm (Amicon) |
| Flow: | 3.5 ml/min. ($R_t$ : UF = 2:1) |
| Reaktionsvolumen: | 5 ml |
| Kreislaufvolumen: | 19 ml |
| Temperatur: | 50 °C |
| Substrat: | Stärke (n. Zulkovsky) |
| Enzymproben-volumen: | 0.1–0.6 ml |

Messanordnung:
Batch-Kreislaufbetrieb, Detektion im Ultrafiltrat durch chem. Nachweis (Nelson-Somogy).

Messlösung:
Amyloglucosidase (Merck, Darmstadt), ca. 7.0 U/ml.

Durchführung:
Die Anlage wurde 10 min. mit Pufferlösung vorgespült. Zu 3.0 ml Pufferlösung (Na-Acetat, pH 4.8) wurden 1.5 ml 1%ige Stärkelösung zugesetzt und für 5 min. im Kreislaufbetrieb umgepumpt, danach wurde aus dem Ultrafiltratstrom eine Probe von 0.1 ml entnommen (Blindwert). Dann wurde eine Enzymmenge von 0.1–0.6 ml zugesetzt und unter fortlaufendem Umpumpen 10 min. lang alle zwei Minuten eine Probe von 0.1 ml entnommen. Die Proben wurden zusammen mit der 0-Probe nach der Methode von Nelson-Somogy auf Glucose getestet.

Auswertung:
Durch Auftragen der gefundenen Konzentrationen an Glucose über der Zeit t in Minuten bekommt man eine Gerade, deren Steigung der Enzymkonzentration entspricht. Mit Glucose als Eichsubstanz wurde eine entsprechende Eichkurve erstellt.

Berechnung: $\frac{\Delta OD_{546} \cdot \text{Verd.faktor}}{10}$

[in U/ml = 1 µMol Glucose pro Minute (pH 4.8; 50°C)]

Eichfaktor: $10 \frac{cm^2}{\mu Mol}$ aus Eichkurve mit Glucose (Nelson-Somogy-Test) bei 546 nm.

Figur 7 zeigt die Konzentrationsabhängigkeit von Amyloglucosidase (chem. Nachweis).

Messwerte:

| Probenvol. (ml) | $\Delta OD_{546}$ | Verd.faktor | U/ml |
|---|---|---|---|
| 0.1 | 0.007 | $9.5 \cdot 10^3$ | 6.65 |
| 0.2 | 0.015 | $4.75 \cdot 10^3$ | 7.13 |
| 0.3 | 0.020 | $3.17 \cdot 10^3$ | 6.34 |
| 0.4 | 0.026 | $2.38 \cdot 10^3$ | 6.19 |
| 0.5 | 0.036 | $1.90 \cdot 10^3$ | 6.84 |
| 0.6 | 0.040 | $1.58 \cdot 10^3$ | 6.32 |

$\bar{X} = 6.58$
S = 0.36 (± 5%)

Abweichung vom Soll-Wert = –6%.

Beispiel 5
Bestimmung einer alkalischen Protease (Fa. Henkel) mit einem synthetischen Substrat
Betriebsbedingungen:

| | |
|---|---|
| UF-Zelle: | CEC-1 (Amicon) |
| Membran: | YM 30, ∅ 90 mm (Amicon) |
| Flow: | 5.7 ml/min. (Rt:UF=1.5:1) |
| Reaktionsvolumen: | 5 ml |
| Kreislaufvolumen: | 13 ml |
| Temperatur: | 50 °C |
| Substrat | Casein-blau (Kupplung von Remazol-Blau an Casein, Fa. Henkel). |
| Enzymproben-volumen: | 1 ml |

Messanordnung:
Batch-Kreislaufbetrieb, Detektion im Ultrafiltrat bei 660 nm.

Messlösung:
Fermenterprobe der Fa. Henkel (Konzentrat), verschiedene Konzentrationen.

Durchführung:
Zu 2.5 ml Pufferlösung wurden 1.5 ml Casein-blau-Lösung (12 mg/ml) in die Reaktionsvorlage gegeben und für ca. 5–10 min. im Kreislauf durch die Messanordnung gepumpt, bis sich ein konstanter Wert am Photometer eingestellt hat. Dann wurde die Reaktion mit 1 ml Enzymlösung gestartet. Nach einer kurzen Totzeit war am angeschlossenen Schreiber der Reaktionsverlauf am kontinuierlichen Anstieg des Photometersignals zu verfolgen.

Die Auswertung erfolgte analog zu Beispiel 1 und 2, durch Anlegen einer Tangente an die Kurve im Bereich der max. Steigung. Der so gefundene Wert für ΔE/min. stellt ein Mass für die Enzymkonzentration dar.

Berechnung: $\frac{\Delta m\ OD_{660} - 4.5}{0.144} = PE/ml$

Eichung:
Unter den gleichen Bedingungen wie oben beschrieben wird eine Eichkurve mit einem Standardenzym (Maxatase, Fa. Henkel) aufgenommen. Die Steigung zur Ermittlung des Eichfaktors ist der Figur 8 zu entnehmen.

Messwerte:

| Pr.-Nr. | PE/ml zugegeben | $\Delta mOD_{666}$ | PE/ml gefunden | % |
|---|---|---|---|---|
| 1 | 50 | 13.5 | 62.5 | 25 |
| 2 | 100 | 19.0 | 100.7 | 1 |
| 3 | 200 | 35.0 | 211.8 | 6 |
| 4 | 300 | 46.0 | 288.2 | 4 |

Mittlere Abweichung vom Soll-Wert ± 9%.

**Patentansprüche**

1. Verfahren zur kinetischen Prozesskontrolle von enzymatischen Stoffumsetzungen, dadurch gekennzeichnet, dass man die Reaktionsmischung, die das zu hydrolysierende Substrat und die hydrolytischen Enzyme enthält, durch eine Ultrafiltrationsmembran in zwei Volumenströme aufteilt, wobei der Ultrafiltrationsstrom die Spaltprodukte und der Retentatstrom das Substrat enthält, und anschliessend die Enzymkonzentration aus der Änderung der Substrat- oder Produktkonzentration im Retentat bzw. Ultrafiltrationsstrom ermittelt.

2. Verfahren nach Anspruch 1, dadurch gekennzeichnet, dass man eine Zelle mit einem Verhältnis des Totvolumens zu wirksamer Membranfläche $V_t/F_m \leq 1$ verwendet.

3. Verfahren nach Anspruch 1 oder 2, dadurch gekennzeichnet, dass man die Veränderung der Produkt- oder Substratkonzentration kontinuierlich mit einem Spektralphotometer, Refraktometer oder Polarimeter misst.

4. Verfahren nach einem der vorhergehenden Ansprüche, dadurch gekennzeichnet, dass man natürliche oder synthetische hochmolekulare Substrate einsetzt.

5. Vorrichtung zur Durchführung des Verfahrens nach einem der vorhergehenden Ansprüche, gekennzeichnet durch ein Reaktionsgefäss (5), das über eine Leitung, in der eine Pumpe (1), sowie Einrichtungen zur Regelung von Druck, Temperatur und Durchfluss (6, 7, 8) angeordnet sind, mit einer Ultrafiltrationszelle (2) verbunden ist, und zwei Leitungen, wobei zumindest in einer ein Detektor (3) angeordnet ist, von der Ultrafiltrationszelle (2) zum Reaktionsgefäss (5).

**Claims**

1. Method for the kinetic process control of enzymatic reactions, characterised in that the reaction mixture, which contains the substrate to be hydrolysed and the hydrolytic enzymes, is divided into two streams by means of an ultrafiltration membrane, the ultrafiltration stream containing the cleavage products and the retentate stream containing the substrate, and the enzyme concentration is subsequently determined from the change in the substrate or product concentration in the retentate or ultrafiltration stream respectively.

2. Method according to claim 1, characterised in that a cell is used which has a ratio of dead volume to effective membrane surface $V_t/F_m$ of $\leq 1$.

3. Method according to claim 1 or 2, characterised in that the change in the product or substrate concentration is measured continuously by means of a spectrophotometer, refractometer or polarimeter.

4. Method according to any one of the preceding claims, characterised in that natural or synthetic substrates of high molecular weight are used.

5. Apparatus for carrying out the method according to any one of the preceding claims, characterised by a reaction vessel (5) which is connected via a line, in which are arranged a pump (1) and devices for regulating pressure, temperature and flow (6, 7, 8), to an ultrafiltration cell (2), and by two lines which lead from the ultrafiltration cell (2) to the reaction vessel (5), a detector (3) being arranged in at least one of these two lines.

**Revendications**

1. Procédé de contrôle cinétique de processus de transformation enzymatiques de substances, caractérisé en ce qu'on divise le mélange réactionnel, qui contient le substrat à hydrolyser et les enzymes à rôle hydrolytique, à l'aide d'une membrane d'ultrafiltration en deux courants, le courant d'ultrafiltration contenant les produits de coupure et le courant du rétentat contenant le substrat, puis l'on détermine la concentration de l'enzyme à partir de la modification de la concentration de substrat ou du produit dans le rétentat ou dans le courant d'ultrafiltrat.

2. Procédé selon la revendication 1, caractérisé en ce qu'on utilise une cellule, ayant un rapport du volume mort à la surface de membrane active $V_t/F_m \leq 1$.

3. Procédé selon la revendication 1 ou 2, caractérisé en ce qu'on mesure continuellement, à l'aide d'un spectrophotomètre, d'un réfractomètre ou d'un polarimètre, la modification de la concentration du produit ou du substrat.

4. Procédé selon l'une des revendications précédentes, caractérisé en ce qu'on utilise des substrats macromoléculaires naturels ou synthétiques.

5. Dispositif pour la mise en œuvre du procédé selon l'une des revendications précédentes, caractérisé en ce qu'il comporte un réacteur (5), qui est relié par l'intermédiaire d'un conduit, auquel soit associés une pompe (1), ainsi que des dispositifs de régulation de la pression, de la température et du débit (6, 7, 8), à une cellule (2) d'ultrafiltration, et deux conduits, à l'un au moins desquels est associé un détecteur (3) reliant la cellule (2) d'ultrafiltration au réacteur (5).

FIG.1

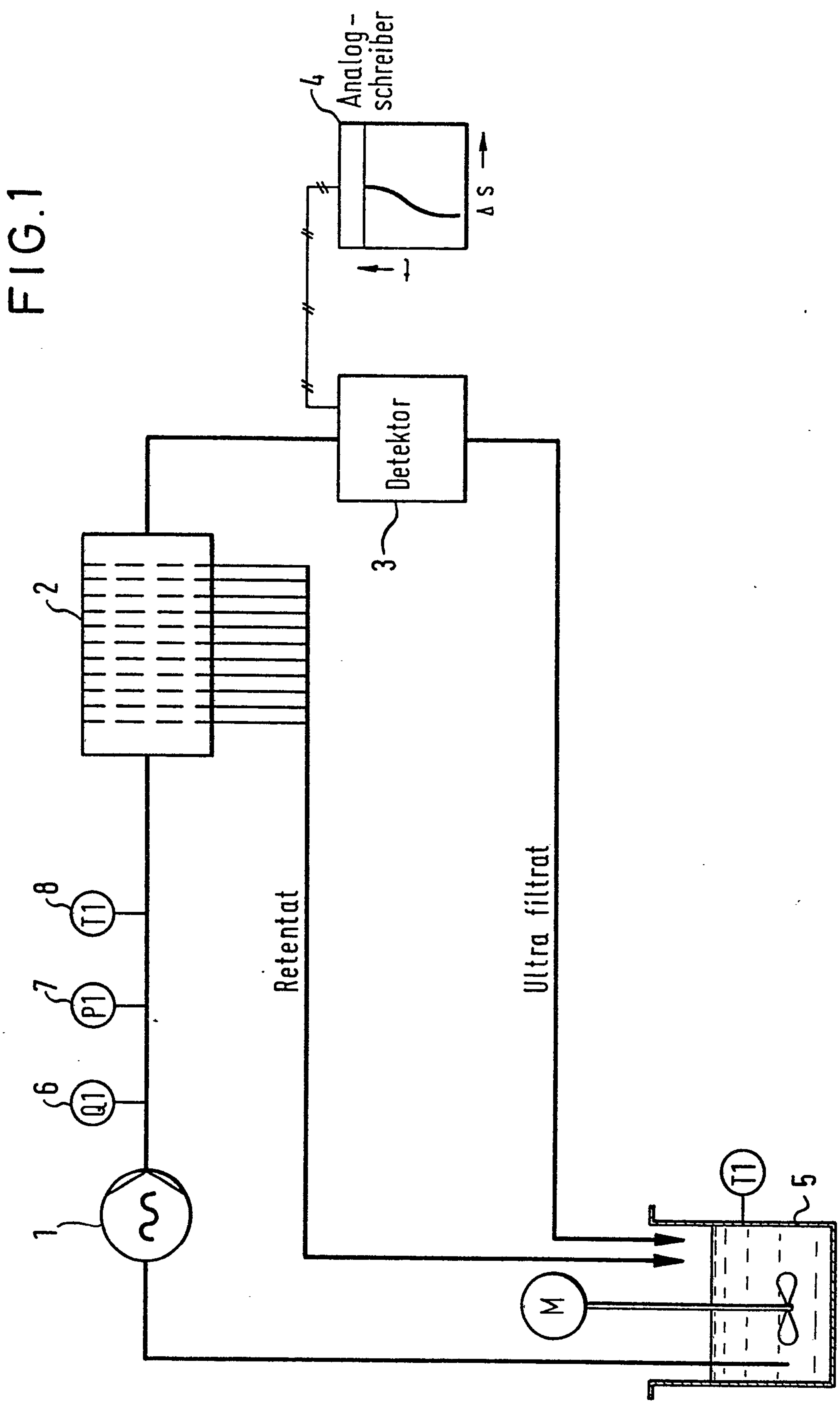

Analog-schreiber
Detektor
Retentat
Ultra filtrat
M
Q1
P1
T1
T1
Δ s
t
1
2
3
4
5
6
7
8

FIG. 2

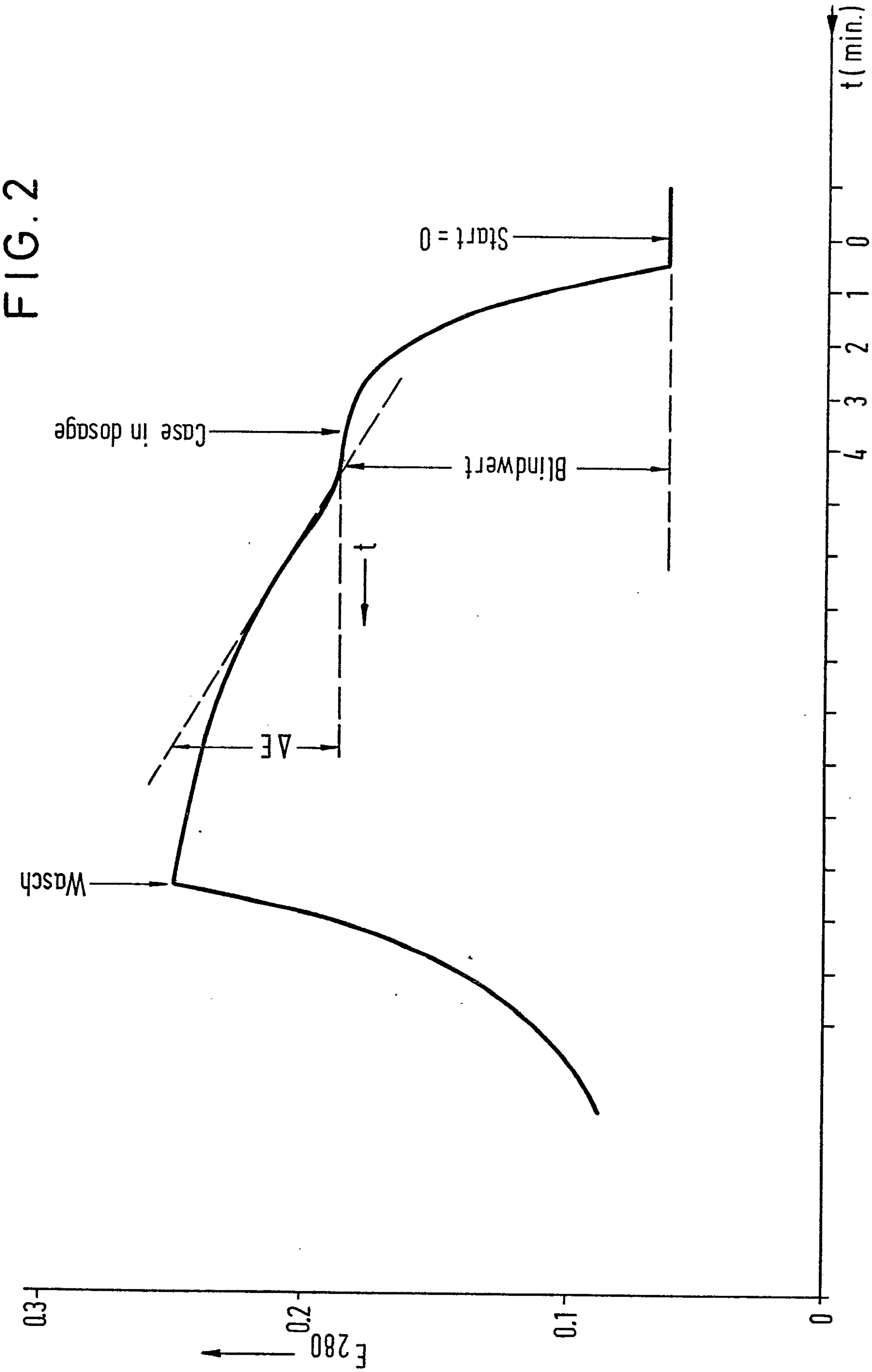

t (min.)
0
1
2
3
4
Start = 0
Case in dosage
Blindwert
t
ΔE
Wasch
0.3
0.2
0.1
0
E 280

FIG. 3

AP 114 - Eichkurve

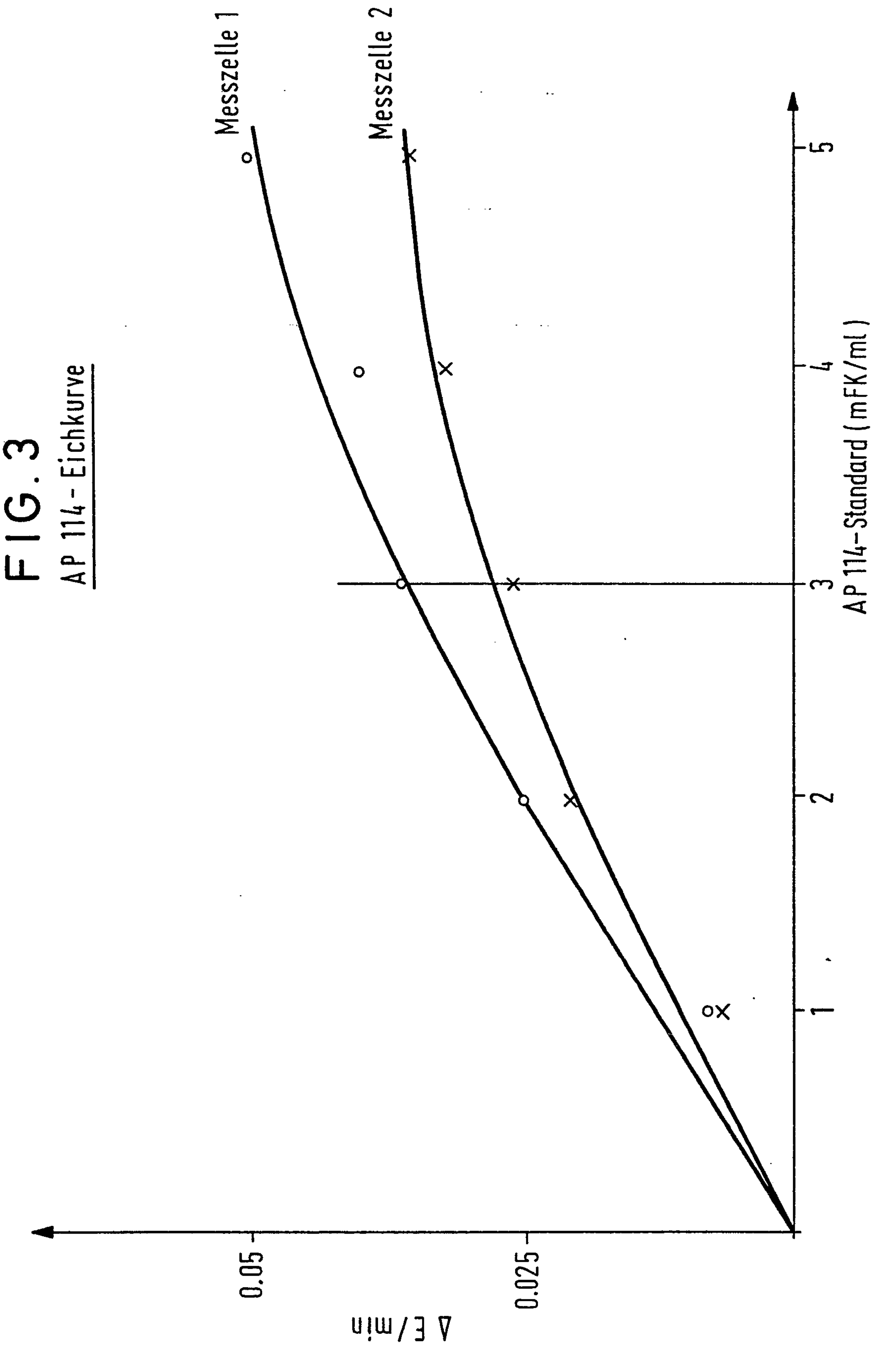

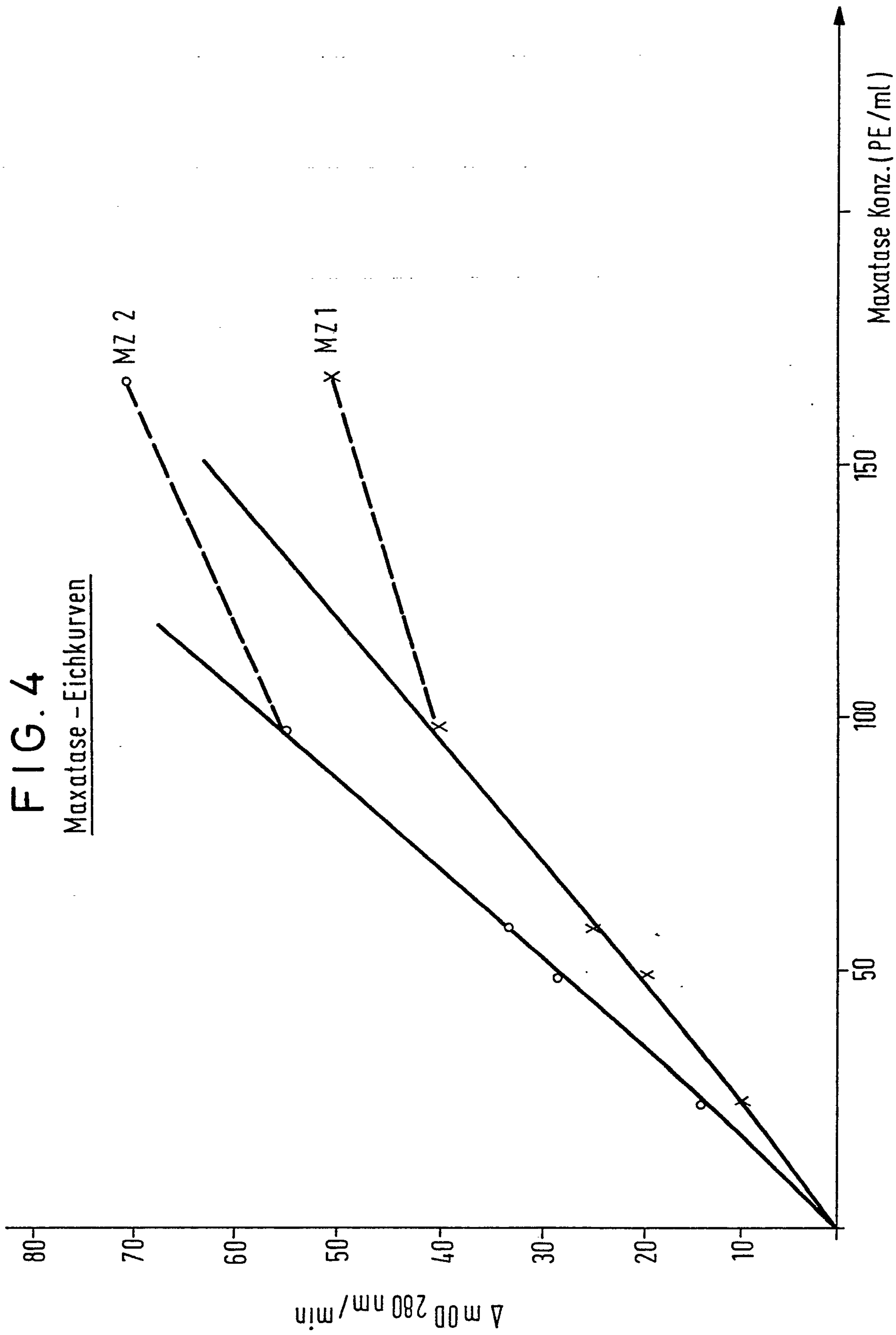


FIG. 4
Maxatase-Eichkurven

FIG. 5

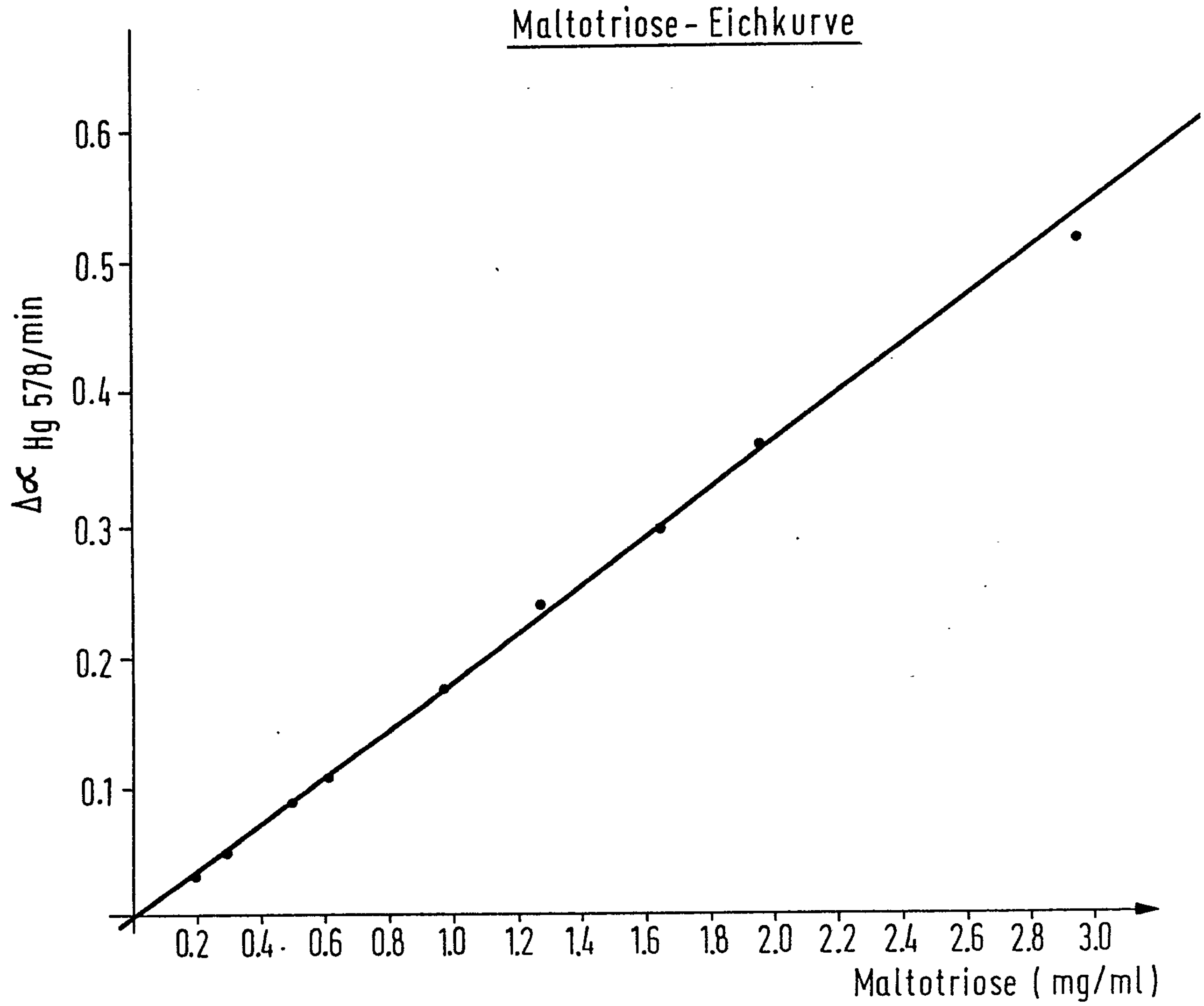
Maltotriose - Eichkurve
Δα Hg 578/min
0.6
0.5
0.4
0.3
0.2
0.1
0.2 0.4 0.6 0.8 1.0 1.2 1.4 1.6 1.8 2.0 2.2 2.4 2.6 2.8 3.0
Maltotriose ( mg/ml )

FIG. 6

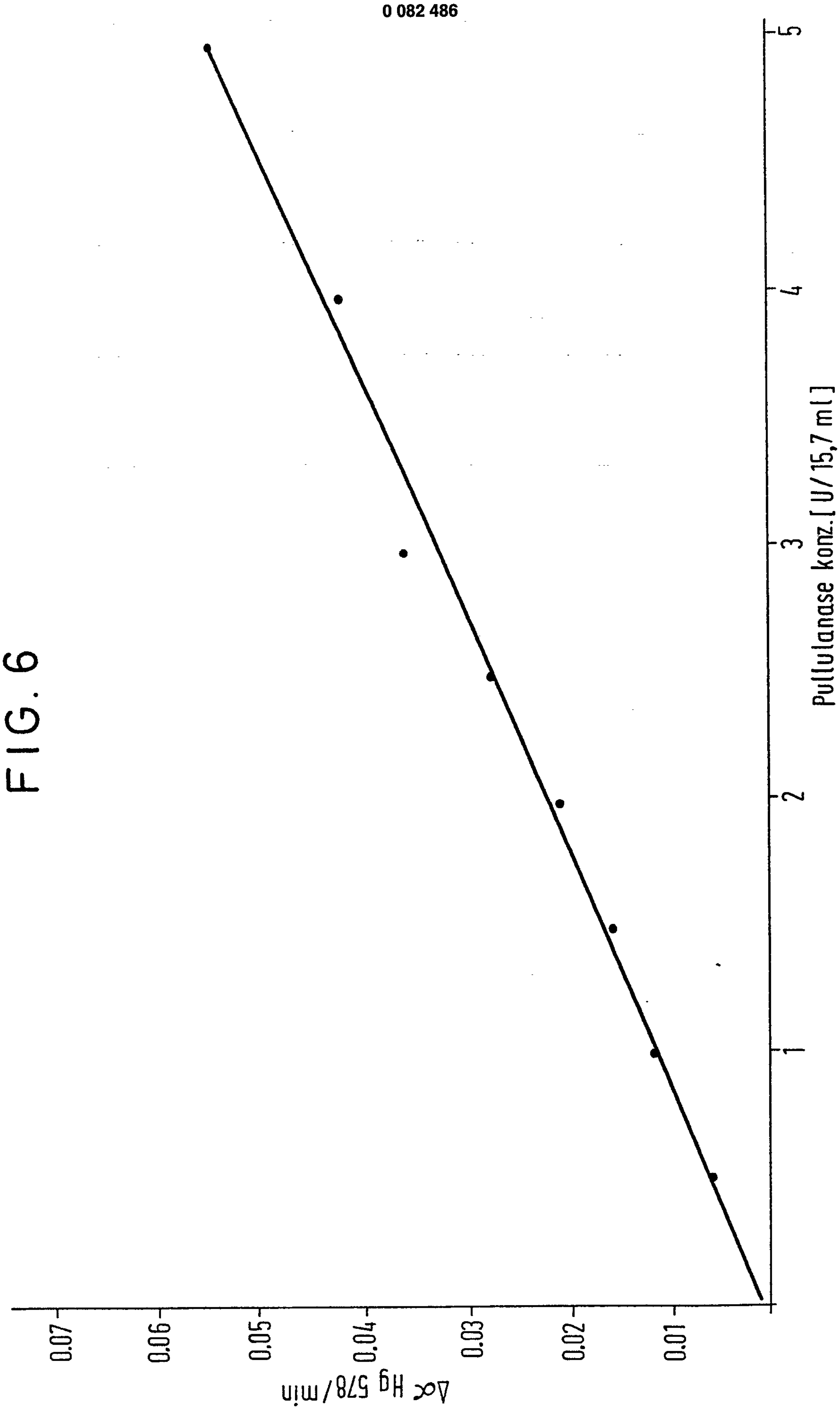

0.07
0.06
0.05
0.04
0.03
0.02
0.01
Δα Hg 578/min
1
2
3
4
5
Pullulanase konz.[ U/15,7 ml ]

FIG. 7

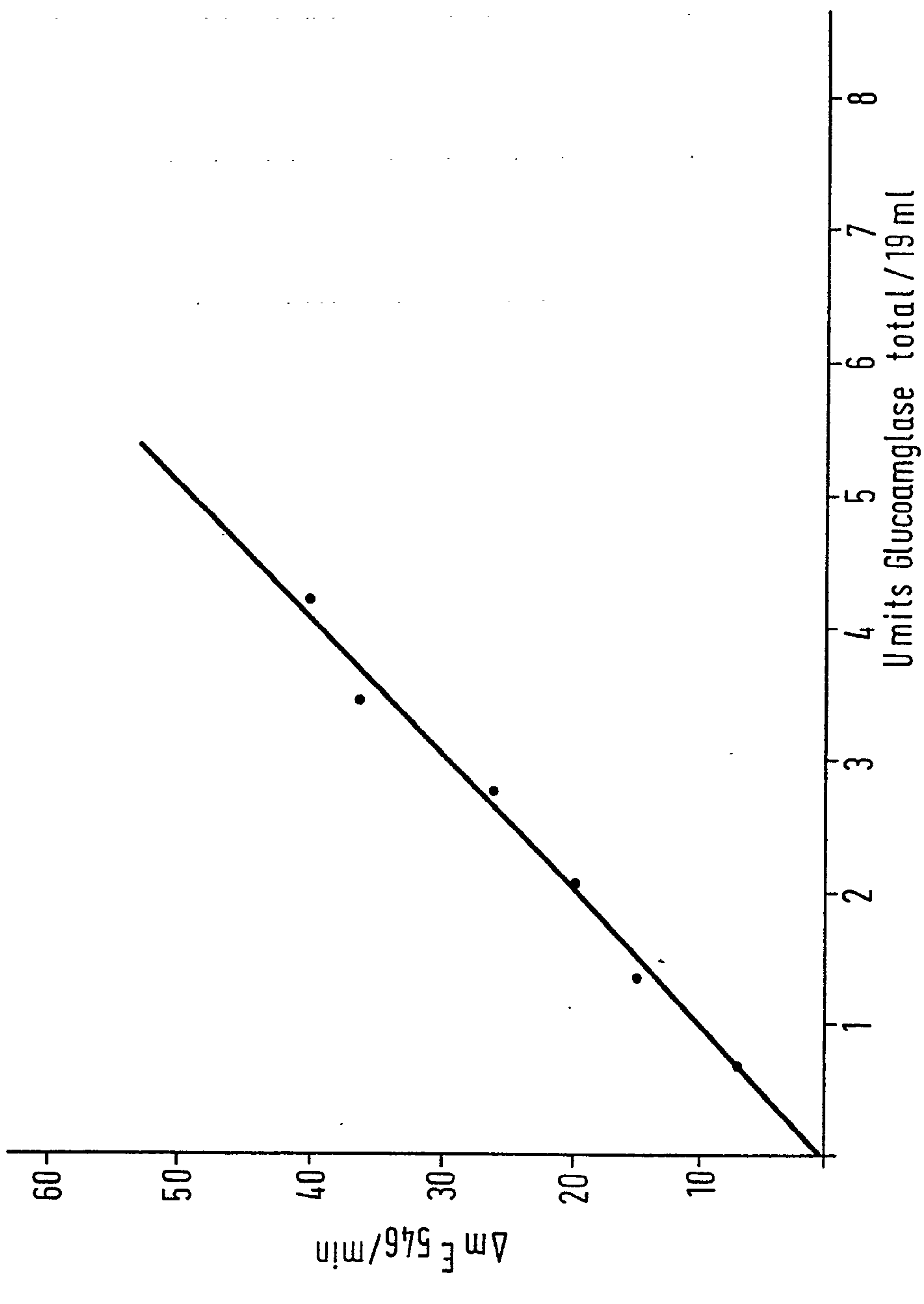
60
50
40
30
20
10
Δm E 546/min
1
2
3
4
5
6
7
8
Umits Glucoamglase total / 19 ml

FIG. 8

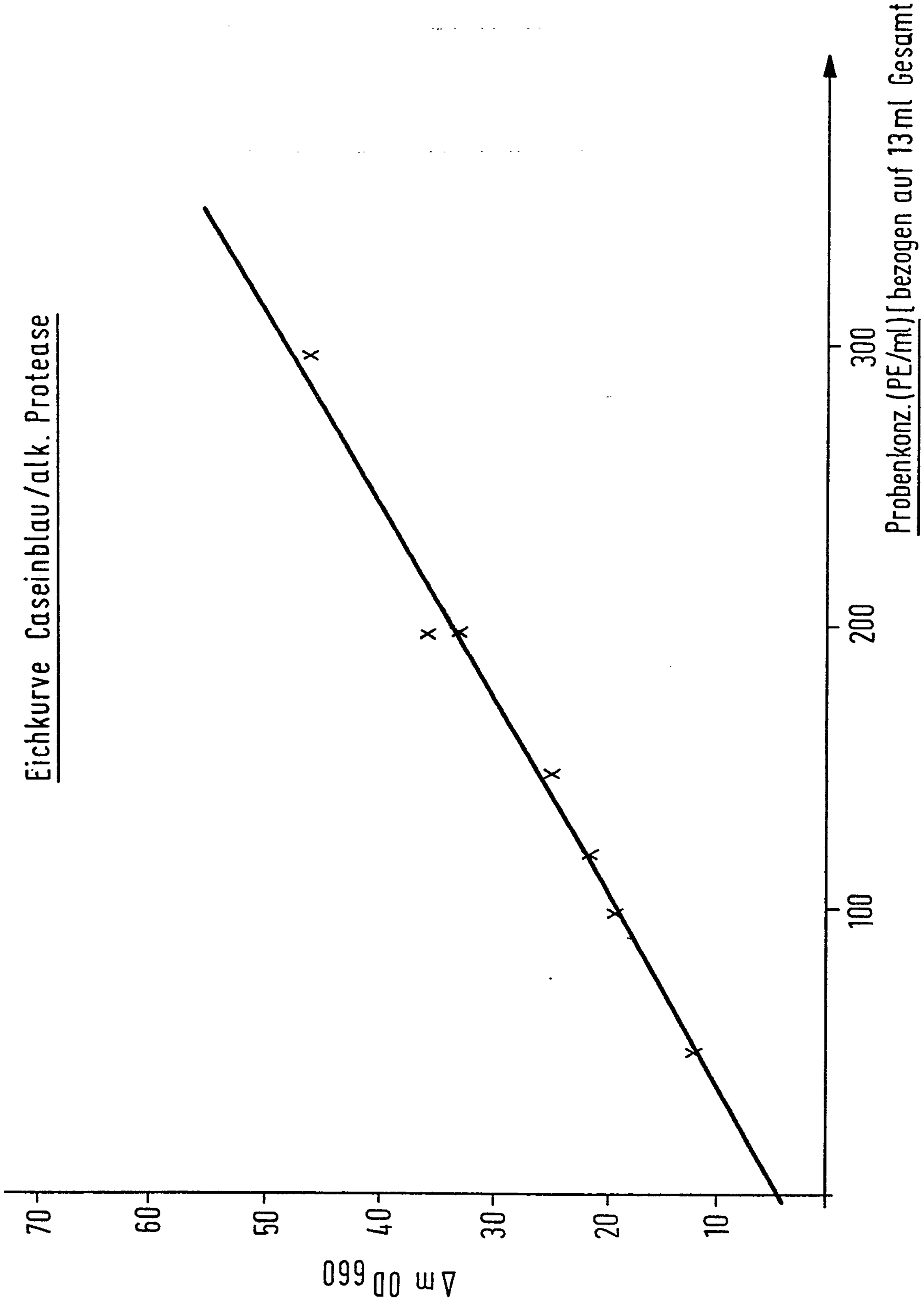
Eichkurve Caseinblau/alk. Protease
Δm OD 660
10
20
30
40
50
60
70
100
200
300
Probenkonz. (PE/ml) [bezogen auf 13 ml Gesamtvol.]